# EUROPÄISCHE PATENTSCHRIFT

(11) **EP 3 373 975 B1**
(45) Veröffentlichungstag und Bekanntmachung des Hinweises auf die Patenterteilung: **09.02.2022**
(21) Anmeldenummer: 16795232.4
(22) Anmeldetag: 14.11.2016
(51) Int. Cl.: A61K 47/36, A61K 31/718, A61K 9/08, A61K 31/185, A61K 31/205, A61K 36/47, A61K 45/06, A61P 7/08, A61P 13/12

(54) **DIALYSELÖSUNG MIT WENIGSTENS EINEM OSMOTIKUM**
DIALYSIS SOLUTION HAVING AT LEAST ONE OSMOTIC AGENT
SOLUTION DE DIALYSE COMPRENANT AU MOINS UN OSMOTIQUE

(30) Priorität: 13.11.2015 DE 102015014699
(43) Veröffentlichungstag der Anmeldung: 19.09.2018
(73) Patentinhaber: Fresenius Medical Care Deutschland GmbH, 61352 Bad Homburg (DE); Friedrich-Schiller-Universität Jena, 07743 Jena (DE)
(72) Erfinder: HEINZE, Thomas, 07743 Jena (DE); HAMPE, Robert, 07743 Jena (DE); BERLICH, Robert, 66606 St. Wendel (DE); FINKLER, Lisa, 66620 Nonnweiler (DE); BURKHART, Jens, Lothar, 66386 St. Ingbert (DE)
(74) Vertreter: Herrmann, Uwe
(86) Internationale Anmeldenummer: PCT/EP2016/001898
(87) Internationale Veröffentlichungsnummer: WO 2017/080675

(56) Entgegenhaltungen:
- EP-A2- 0 602 585
- WO-A1-2004/022602
- KR-A- 20140 092 593
- KR-A- 20140 092 593
- US-A1- 2012 295 873

## Beschreibung

Die vorliegende Erfindung betrifft eine Dialyselösung mit wenigstens einem Osmotikum.

Dialyselösungen, wie sie beispielsweise bei der Peritonealdialyse, Hämodialyse, Hämodiafiltration etc. eingesetzt werden, sind in zahlreichen unterschiedlichen Zusammensetzungen bekannt.

So offenbart beispielsweise die DE 10 2004 023 828 A1 eine Lösung für eine Peritonealdialyse, die neben Elektrolyten ein Osmotikum in Form von Glucose enthält.

Die als Osmotikum dienende Glucose führt dazu, dass der Transport von Wasser über die Membran beschleunigt und somit die Ultrafiltrationsrate verbessert wird. Ein weiteres bekanntes Osmotikum ist Icodextrin, bei dem es sich um ein stärkebasiertes, verzweigtes Glucosepolymer handelt.

Ferner offenbart die KR 2014 0092593 Stärkesulfat als Osmotikum.

Der vorliegenden Erfindung liegt die Aufgabe zugrunde, eine Dialyselösung dahingehend weiterzubilden, dass deren Ultrafiltrationsleistung gegenüber bekannten Dialyselösungen gesteigert ist.

Diese Aufgabe wird durch eine Dialyselösung mit den Merkmalen des Anspruchs 1 gelöst. Danach ist vorgesehen, dass es sich bei dem Osmotikum um um ein Polysaccharid handelt, das mit 2-Sulfoethylgruppen modifiziert ist.

Es hat sich überraschenderweise gezeigt, dass Polysaccharide, die mit 2-Sulfoethylgruppen modifiziert sind, eine sehr hohe Wirksamkeit bei der Verwendung als osmotische Agenzien aufweisen.

Die Modifikation erfolgt an den freien Hydroxylgruppen des Polysaccharids. Die 2-Sulfoethylgruppen haben die chemische Formel -CH₂-CH₂-S(=O)(=O)OR, wobei R Wasserstoff oder einem Kohlenwasserstoffrest mit beispielsweise 1 bis 10 Kohlenstoffatomen darstellt und vorzugsweise Wasserstoff ist.

In einer weiteren Ausführungsform ist R ein Kation, beispielsweise Natrium oder Kalium, so dass die Sulfoethylstärke als Salz, vorzugsweise als Natriumsalz, vorliegt.

In einer Ausführungsform handelt es sich bei dem modifizierten Polysaccharid um eine 2-Sulfoethylstärke.

Als Ausgangsmaterialien für die Herstellung der 2-Sulfoethylstärke sind Stärken verschiedener Quelle (z.B. aus Kartoffel, Mais, Maniok (Tapioka), Reis, Erbse, Weizen und weitere Getreidearten) sowie spezielle Stärketypen wie Hylon VII, Amiocapowder oder Wachsmaisstärke denkbar.

Durch die Wahl der Reaktionsbedingungen ist es möglich, eine bevorzugte Substitution an der 2er oder der 6er Position zu erreichen. Die 3er Position ist typischerweise schwächer substituiert als sowohl die 2er als auch die 6er Position.

In einer bevozugten Ausführung der Erfindung erfolgt die Herstellung des modifizierten Polysaccharids durch die Umsetzung von Stärke mit Natriumvinylsulfonat.

In einer weiteren bevorzugten Ausgestaltung der Erfindung erfolgt die Reduktion der Stärke mit Natriumborhydrid vor der Sulfoethylierung.

In einer Ausführungsform weist das modifizierte Polysaccharid einen Substitutionsgrad von zwischen 0,05 und 1,0 und vorzugsweise von zwischen 0,2 und 0,5 auf. Der Substitutionsgrad ist definiert als die durchschnittliche Anzahl an Substituenten per Wiederholungseinheit des Polymers.

In einer Ausführungsform liegt die molare Masse des dem modifizierten Polysaccharid zugrundeliegenden unmodifizierten Polysaccharids zwischen 1.000 und 50.000 g/mol und vorzugsweise zwischen 1.000 und 20.000 g/mol.

Die molare Masse der unmodifizierten Sulfoethylstärke liegt vorzugweise zwischen 1.000 und 50.000 g/mol, und besonders bevorzugt zwischen 1.000 und 20.000 g/mol.

In einer Ausführungsform enthält die Dialyselösung außer dem modifizierten Polysaccharid kein weiteres Osmotikum wie beispielsweise unmodifizierte Stärke, Icodextrin oder Glukose.

Alternativ sind auch Mischungen aus dem modifizierten Polysaccharid und Additiven und/oder weiteren Osmotika wie beispielsweise unmodifizierte Stärke, Icodextrin , L-Carnitin, Dipeptiven, Taurin oder Glukose oder eine Kombination zweier oder mehrerer dieser Komponenten denkbar.

In einer Ausführungsform weist die Dialyselösung genau eine Art des modifizierten Polysaccharids auf. Alternativ sind auch Mischungen aus mehreren derartiger Polysaccharide denkbar.

In einer Ausführungsform ist das modifizierte Polysaccharid vollständig wasserlöslich.

In einer Ausführungsform enthält die Dialyselösung ferner Elektrolyte und ein Puffersystem. Geeignete Elektrolyte umfassen Natrium-, Kalium-, Calcium-, Magnesium- und/oder Chloridionen. Geeignete Puffersysteme umfassen Lactatpuffer, Hydrogencarbonatpuffer oder eine Kombination daraus. Das Puffersystem dient zur Einstellung eines physiologischen pH-Wertes.

Der pH-Wert der Dialyselösung liegt vorzugsweise im Bereich zwischen 5,0 und 8,0.

Handelt es sich um ein Einkammerbeutelsystem liegt der pH-Wert vorzugsweise zwischen 5,0 und 8,0, besonders bevorzugt zwischen 5,5 und 6,5.

Handelt es sich um ein Zweikammerbeutelsystem liegt der Misch pH-Wert (nach dem Mischen der Teillösungen) vorzugsweise zwischen 5,0 und 8,0, und besonders bevorzugt zwischen 6,5 und 7,5. Der pH-Wert der sauren Teillösung liegt vorzugsweise zwischen 3,0 und 5,0 und der pH-Wert der basischen Teillösung liegt vorzugsweise zwischen 7,0 und 9,0.

In einer Ausführungsform liegen die Elektrolyte, soweit vorhanden und unabhängig voneinander, in den folgenden Konzentrationen in der Dialyselösung vor (Angaben in mmol/l):

| | |
|---|---|
| Natriumionen | 125-150 |
| Kaliumionen | 0-4,5 |
| Calciumionen | 0-2,5 |
| Magnesiumionen | 0-5 |
| Chloridionen | 90-120 |
| Lactat/Milchsäure/(Hydrogen)Carbonat/CO₂ | 30-60 |

Die erfindungsgemäße Dialyselösung dient vorzugsweise der Anwendung in der Peritonealdialyse. Alternativ ist auch eine Anwendung der erfindungsgemäßen Lösung in der Hämodialyse oder der Hämodiafiltration denkbar.

Die vorliegende Erfindung betrifft des weiteren ein Verfahren zur Herstellung eines Osmotikums einer Dialyselösung durch Modifikation eines Polysaccharids mit 2-Sulfoethylgruppen. Sämtliche der oben genannten Merkmale können auch Bestandteil des Verfahrens sein, d.h. der sich auf die erfindungsgemäße Dialyselösung beziehende Offenbarungsgehalt ist entsprechend auch Offenbarungsgehalt des erfindungsgemäßen Verfahrens.

Weitere Einzelheiten und Vorteile werden anhand der nachfolgend beschriebenen Figuren und Ausführungsbeispiele erläutert. In den Figuren zeigen:
Figur 1: schematische Darstellungen von mit der erfindungsgemäßen Dialyselösung gefüllten Schlauches zu unterschiedlichen Zeitpunkten;
Figur 2: Verläufe der Volumenzunahme für mit unterschiedlichen Dialyselösung gefüllte Schläuche über die Zeit;
Figur 3: ein Balkendiagramm über die Volumenzunahme für mit unterschiedlichen Dialyselösung gefüllte Schläuche nach 24 Stunden und
Figuren 4 - 11: 1H-NMR und ¹³C-NMR Spektren von Sulphoethylstärke gemäß der Beispiele 1 bis 4.

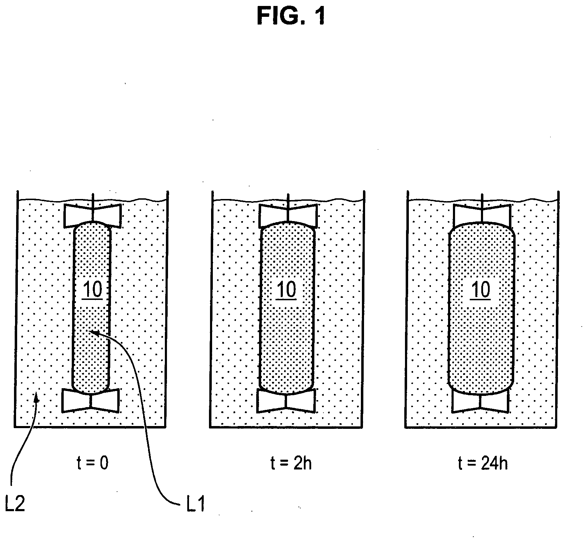

Figur 1 zeigt einen Schlauch 10, dessen Wandung durch eine semipermeable Membran gebildet wird. Bei der Membran kann es sich beispielsweise um regenerierte Cellulose handeln.

In dem Inneren des Schlauches 10 befindet sich eine Dialyselösung L1, die ein Osmotikum enthält. Der Schlauch 10 befindet sich in einer Lösung L2, die dieselbe Zusammensetzung aufweist wie die Lösung L1 in dem Schlauch 10 mit dem einzigen Unterschied, dass die Lösung L2 kein Osmotikum aufweist.

Figur 1, linke Darstellung zeigt die Anordnung zum Zeitpunkt t=0, d.h. zu dem Versuchsbeginn, zu dem der gefüllte Schlauch 10 in die Lösung L2 eingesetzt wurde.

Figur 1, mittlere Darstellung zeigt die Anordnung nach zweistündiger Einwirkdauer (t=2h) und Figur 1, rechte Darstellung zeigt die Anordnung nach 24-stündiger Einwirkdauer (t=24 h).

Wie dies aus einem Vergleich der Abbildungen gemäß Figur 1 hervorgeht, ist aufgrund der osmotischen Wirkung der Lösung L1 Wasser in den Schlauch eingeströmt, womit sich dessen Volumen über die Zeit entsprechend vergrößert.

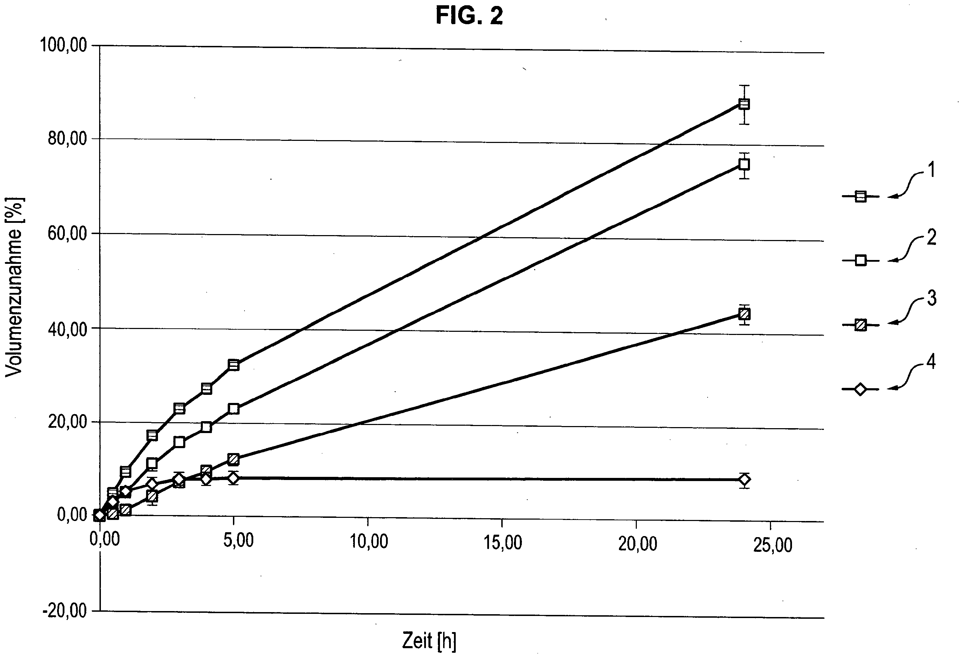

Figur 2 zeigt die Volumenzunahme in % (ausgehend von dem Versuchsstart bei t=0) über die Zeit bei einem weiteren Schlauchversuch für Lösungen mit unterschiedlichen Osmotika. Dabei weisen diejenigen Lösungen, deren Verläufe mit den Bezugszeichen 1 und 2 versehen sind, als einziges Osmotikum 5 % (w/v) 2-Sulfoethylstärke mit einem durchschnittlichen Substitutionsgrad DS von 0,46 (Lösung 1) und 0,20 (Lösung 2), jeweils hergestellt aus Tapiokastärke (zahlenmittlere Molmasse, Mn = 3321 g/mol), auf.

Die Bezugszeichen 3 und 4 betreffen Lösungen mit 5 % (w/v) Glucose (Lösung 4) und mit 5 % (w/v) Icodextrin (Lösung 3).

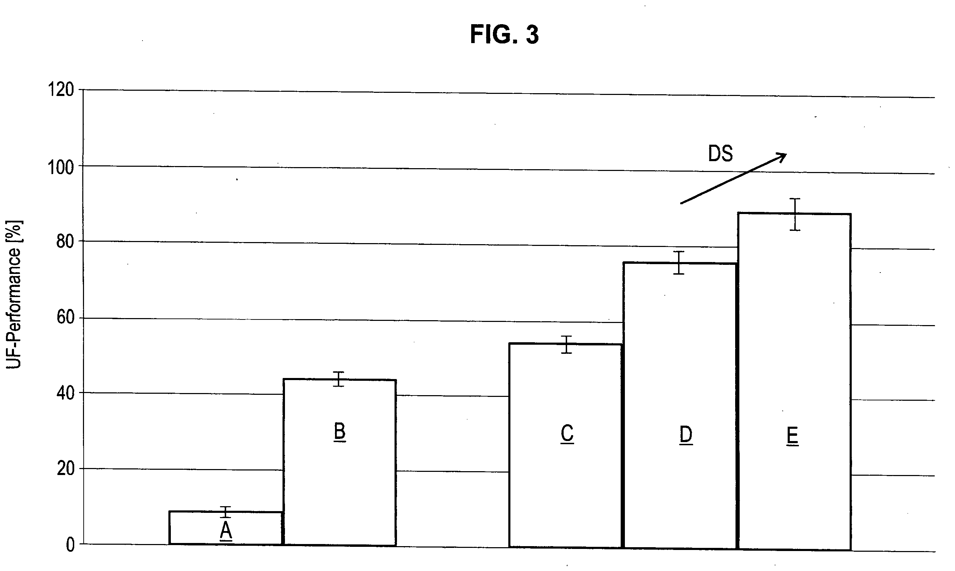

Eine weitere Auswertung der osmotischen Wirkung bekannter und erfindungsgemäßer Osmotika ist in Figur 3 gezeigt, wobei Figur 3 auf der Ordinate den Volumenzuwachs, d.h. die UF-Performance (UF=Ultrafiltration) des Schlauches 10 nach 24-stündiger Einwirkdauer zeigt. Der Wert 0 % bedeutet, dass sich gegenüber dem Versuchsbeginn (t=0) keine Volumenänderung ergeben hat, der Wert 100 % bedeutet gegenüber dem Ausgangszustand bei t=0 eine Volumenverdopplung.

Die Versuchsbedingungen waren für beide Figuren 2 und 3 identisch.

Das Bezugszeichen A zeigt das Ergebnis bei Einsatz von 5 % (w/v) Glucose und verdeutlicht, dass nach 24-stündiger Einwirkzeit eine Volumenzunahme um 10% erfolgt ist. Das Bezugszeichen B zeigt das Ergebnis bei der Verwendung einer 5 % (w/v) Icodextrinlösung, wobei nach 24-stündiger Einwirkdauer eine Volumenzunahme von 40% erfolgt ist. Das Bezugszeichen C zeigt das Ergebnis bei Einsatz von 5 % (w/v) Tapiokastärke (Mn = 3321 g/mol), wobei nach 24-stündiger Einwirkdauer eine Volumenzunahme von gut 50% erfolgt ist.

Die Bezugszeichen D und E zeigen das Ergebnis für Dialyselösungen gemäß der vorliegenden Erfindung, wobei als einziges Osmotikum 5 % (w/v) 2-Sulfoethylstärke mit einem durchschnittlichen Substitutionsgrad DS von 0,46 (E) und 0,20 (D), jeweils hergestellt aus Tapiokastärke (zahlenmittlere Molmasse, Mn = 3321 g/mol). wie im Zusammenhang mit Figur 2 beschrieben zum Einsatz kommen.

Aus Figur 3 wird deutlich, dass bei dem Einsatz der erfindungsgemäßen Osmotika ein Volumenzuwachs von bis zu 90% erzielt wird, was deutlich über dem liegt, was mit bekannten Osmotika erreicht wird.

Die Versuchsbedingungen für die Ergebnisse gemäß Figur 2 und 3 sind wie folgt: In einen Schlauch mit einer semipermeablen Schlauchwandung aus regenerierter Cellulose (MWCO: 1000 Da, Fa. Carl Roth) wurde ein Füllvolumen von 10 ml einer Flüssigkeit eingefüllt. Diese Flüssigkeit besteht aus einer wässrigen Lösung des entsprechenden Osmotikums mit einer Konzentration des Osmotikums von 5 %

(w/v), wobei weitere Inhaltsstoffe durch Ca²⁺ in einer Konzentration von 1 mmol/l, Mg²⁺ in einer Konzentration von 0,5 mmol/l, Na⁺ in einer Konzentration von 138 mmol/l, Cl⁻ in einer Konzentration von 106 mmol/l und Lactat in einer Konzentration von 35 mmol/l vorliegen.

Dieser gefüllte Schlauch wurde bei einer Temperatur von 38 °C in einem Bad derselben Versuchslösung allerdings ohne osmotisches Agens für 24 Stunden unter Bewegung gelagert.

Zu verschiedenen Zeitpunkten wurde die Volumenzunahme des Füllvolumens des Schlauches ermittelt, die die osmotische Wirkung des Agens wiederspiegelt. Wie dies aus Figur 2 ersichtlich ist, wurden die osmotischen Agenzien gemäß der vorliegenden Erfindung mit bekanntem osmotischen Agenzien in Form von Glucose und Icodextrin verglichen.

Figur 2 zeigt, dass nach 24-stündiger Einwirkdauer die Volumenzunahme des Schlauches für sämtliche Dialyselösungen, die 2-Sulfoethylstärke enthalten, über 70 % liegt. Als Maximalwert wird eine Volumenzunahme von ca. 95 % nach 24 Stunden erreicht.

Demgegenüber liegen die Endwerte nach 24 h für Icodextrin bei gut 40 % und von Glucose bei ca. 10 %.

Die erfindungsgemäßen Osmotika zeigen nicht nur eine erhöhte Ultrafiltrationseffizienz nach 24 Stunden, sondern gegenüber Icodextrin auch einen höheren Wert bei geringen Verweildauern.

Während die Volumenzunahme bei Icodextrin im Wesentlichen linear verläuft, ist bei den Dialyselösungen, die 2-Sulfoethylstärke enthalten, ein vergleichsweise steiler Anstieg zu erkennen, der dann, bei höheren Verweilzeiten etwas abflacht und in einen im Wesentlichen linearen Verlauf übergeht.

Der Anstieg des Schlauchvolumens bei kleinen Verweilzeiten ist bei dem Einsatz von 2-Sulfoethylstärke vergleichbar mit dem von Glucose. Bei höheren Werten ist jedoch der Volumenanstieg bei Glucose als Osmotikum weitaus geringer und bleibt ab einer Verweildauer von ca. drei Stunden konstant, wie dies aus Figur 2 hervorgeht.

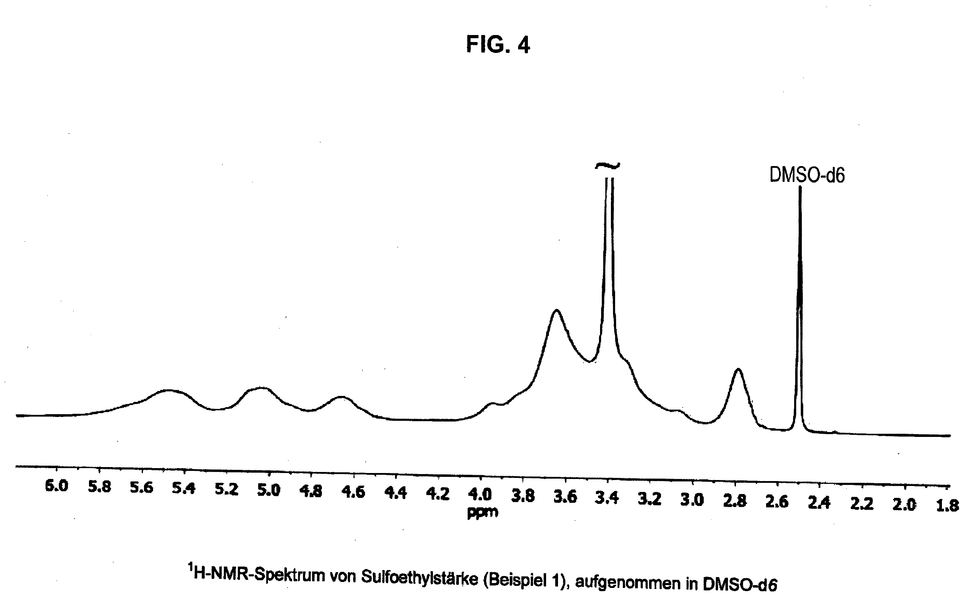

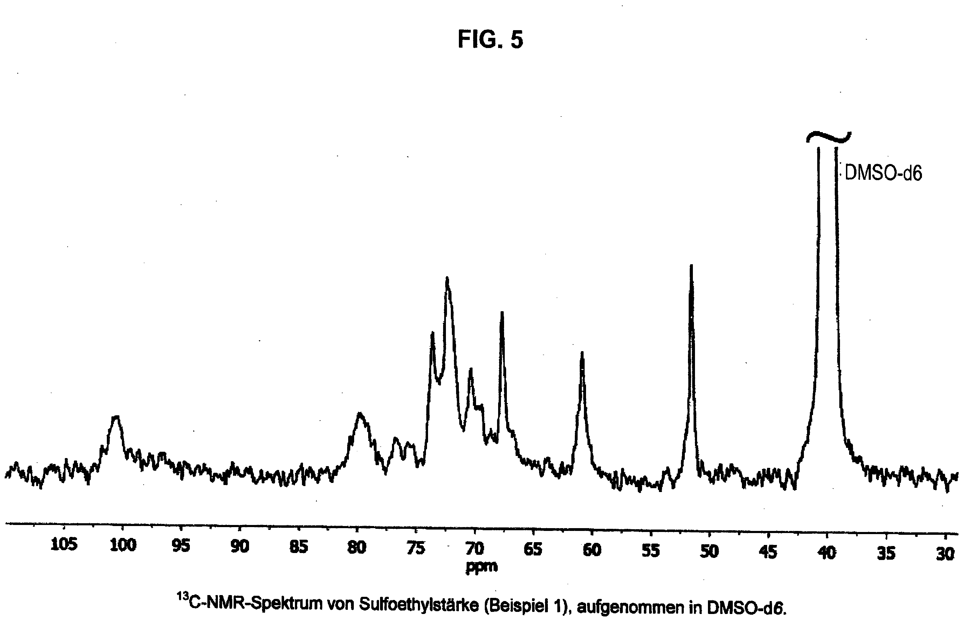

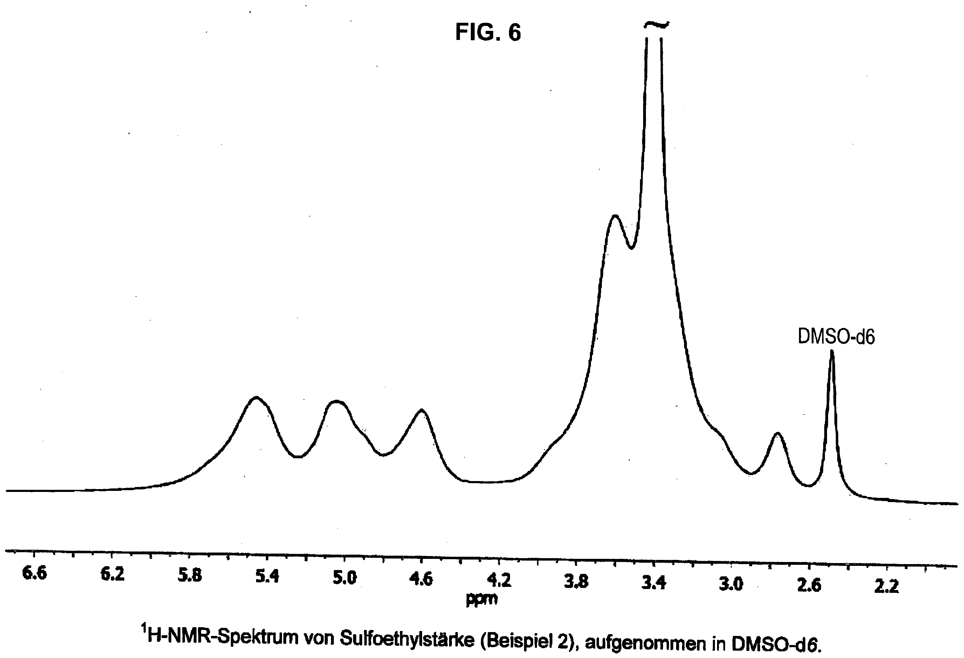

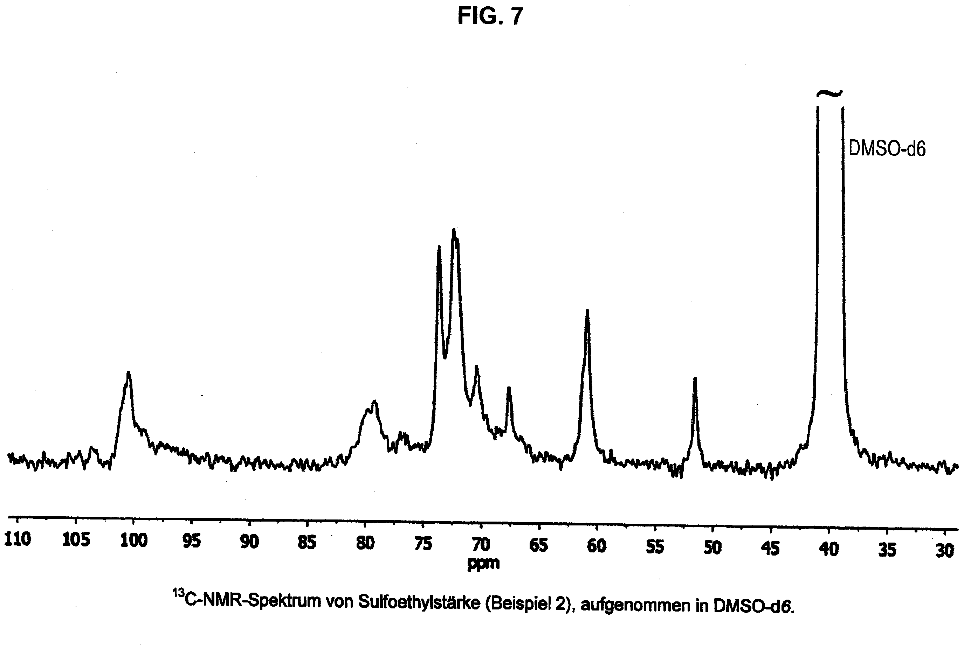

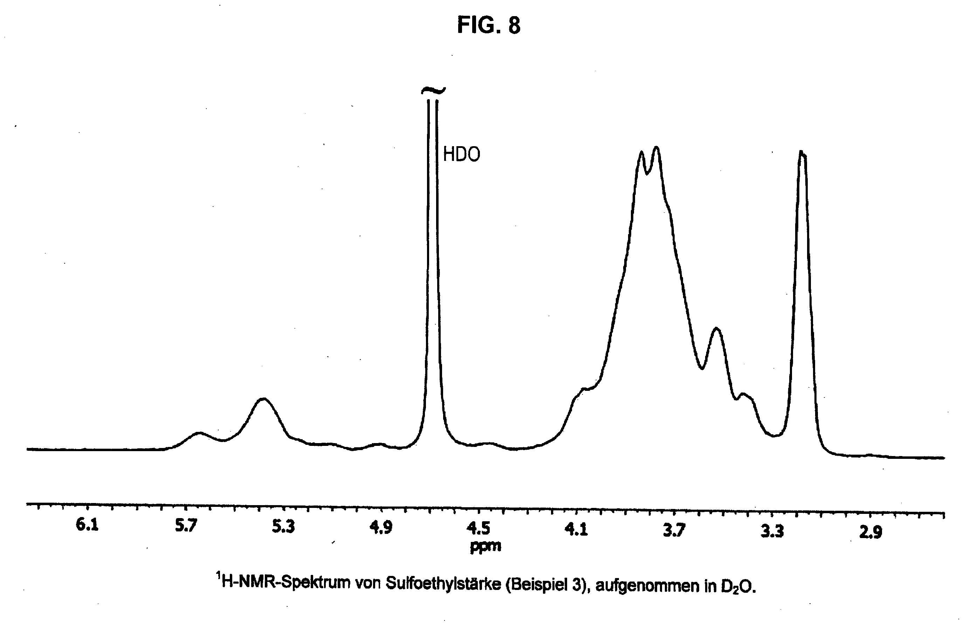

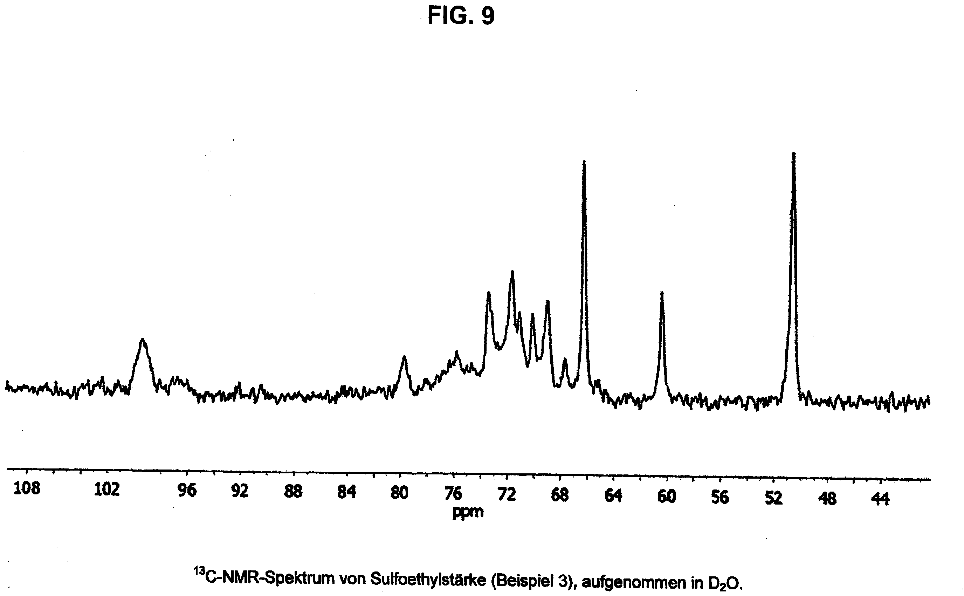

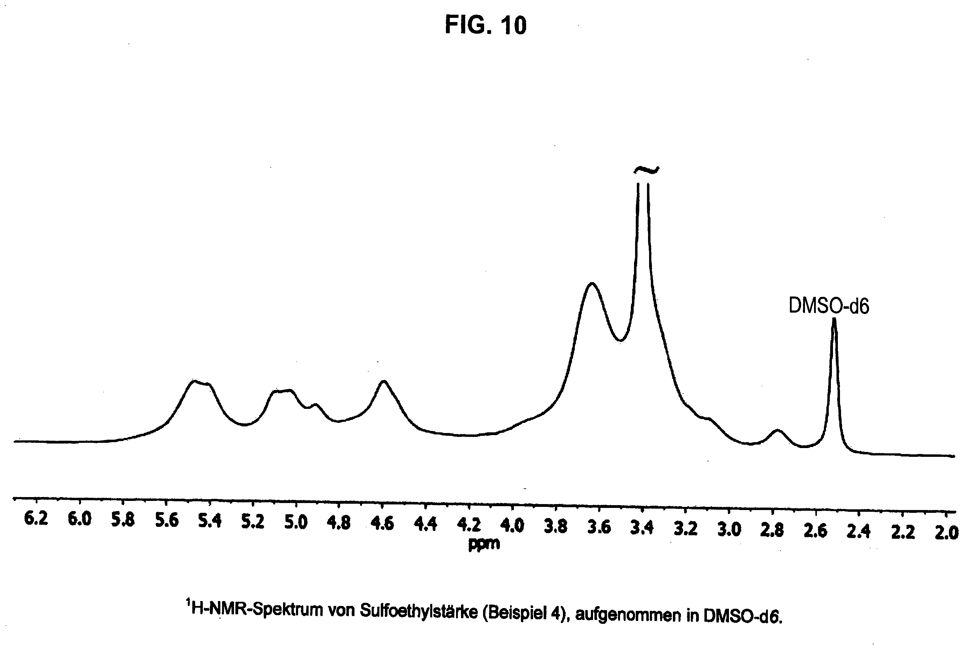

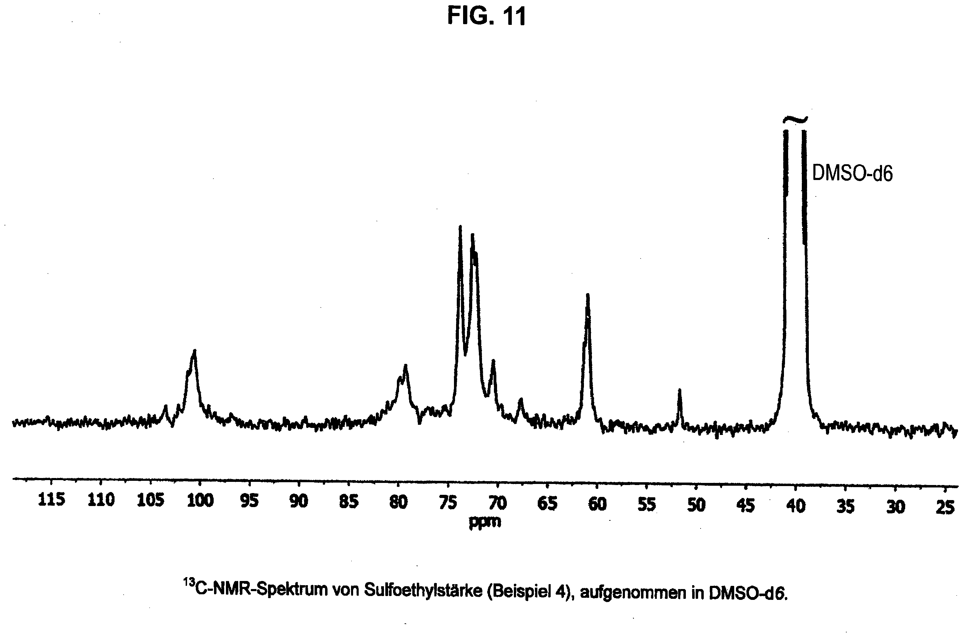

Im Folgenden werden einige Ausführungsbeispiele zur Durchführung der Erfindung beschrieben:
Beispiel 1: 40,0 g abgebaute Tapiokastärke (^{M̅}ₙ = 3.321g/mol) in 480 mL Isopropanol werden in Stickstoffatmosphäre unter Rühren mit 77,1 g 25 %iger (w/w) wässriger Natriumvinylsulfonatlösung (0,6 mol/mol Anhydroglucoseeinheit, AGE), in der 14,82 g NaOH gelöst wurden, versetzt. Die Reaktionsmischung wird auf 80 °C erhitzt, 1 h bei dieser Temperatur gerührt und anschließend werden erneut 77,1 g der Natriumvinylsulfonatlösung (0,6 mol/mol AGE) zugefügt. Nach weiteren 3 h Reaktionszeit bei 80 °C wird das Gemisch auf Raumtemperatur abgekühlt und mit Essigsäure neutralisiert. Das Produkt wird in 4 L Methanol gefällt, filtriert, fünfmal mit 1 L Methanol gewaschen und zweimal aus 150 mL Wasser in 1,5 L Methanol umgefällt. Es wird in 300 mL Wasser gelöst, die Polymerlösung filtriert und gefriergetrocknet. Der Strukturbeleg erfolgte durch ¹H- und ¹³C-NMR-Spektroskopie (Abbildungen 4 und 5) sowie Elementaranalyse. Durchschnittlicher Substitutionsgrad (DS, bestimmt mittels Elementaranalyse): 0,46.
Beispiel 2: Gemäß Beispiel 1 werden 40,0 g abgebaute Tapiokastärke (^{M̅}ₙ = 3.321g/mol) mit insgesamt 64,28 g 25 %iger wässriger Natriumvinylsulfonatlösung (0,5 mol/mol AGE) und 7,41 g NaOH umgesetzt. Das Rohprodukt wird vom Reaktionsmedium dekantiert, dreimal mit je 500 mL Methanol gewaschen und dreimal aus 300 mL Wasser in 3 L Methanol umgefällt. Nach Lösen in 300 mL Wasser wird die Lösung filtriert und gefriergetrocknet. ¹H- und ¹³C-NMR-Spektren bestätigen die Struktur (Abbildungen 6 und 7).
   DS (bestimmt mittels Elementaranalyse): 0,20.
Beispiel 3: Gemäß Beispiel 1 werden 40,0 g abgebaute Tapiokastärke (^{M̅}ₙ = 3.321g/mol) mit insgesamt 385,64 g 25 %iger (w/w) wässriger Natriumvinylsulfonatlösung (3 mol/mol AGE) und 44,46 g NaOH umgesetzt. Die erhaltene Sulfoethylstärke wird nach Isolierung ein zweites Mal gemäß Beispiel 1 umgesetzt (1,2 mol Natriumvinylsulfonat/ mol AGE) und gereinigt. ¹H- und ¹³C-NMR-Spektren bestätigen die Struktur (Abbildungen 8 und 9).
   DS (bestimmt mittels Elementaranalyse): 0,68.
Beispiel 4: 30,0 g abgebaute Tapiokastärke (^{M̅}ₙ = 3.321g/mol) bei 80 °C werden in 270 mL Wasser gelöst und die Lösung mit NaHCO₃ auf pH 8 eingestellt. Nach Abkühlung auf 60 °C wird portionsweise 1,74 g Natriumborhydrid (0,25 mol/mol AGE) zugefügt und die Lösung 24 h gerührt. Durch Zugabe von 10 mL Aceton wird das überschüssige Natriumborhydrid zerstört. Nach Einengung der Lösung im Vakuum und Filtration wird die Probe gegen Wasser dialysiert (Membran mit Molecular weight cut-off von 2500 g/mol) und anschließend gefriergetrocknet.
   Gemäß Beispiel 2 werden 25,0 g der mit Natriumborhydrid behandelten Stärke mit insgesamt 40 g 25 %iger (w/w) wässriger Natriumvinylsulfonatlösung (0,5 mol/mol AGE) und 4,63 g NaOH umgesetzt und isoliert. ¹H- und ¹³C-NMR-Spektren bestätigen die Struktur (Abbildungen 10 und 11).
   DS (bestimmt mittels Elementaranalyse): 0,09.

## Patentansprüche

1. Dialyselösung mit wenigstens einem Osmotikum,
**dadurch gekennzeichnet,**
**dass** es sich bei dem Osmotikum um ein Polysaccharid handelt, das mit 2-Sulfoethylgruppen modifiziert ist.

2. Dialyselösung nach Anspruch 1, **dadurch gekennzeichnet, dass** es sich bei dem modifizierten Polysaccharid um eine 2-Sulfoethylstärke handelt.

3. Dialyselösung nach einem der vorhergehenden Ansprüche, **dadurch gekennzeichnet, dass** das modifizierte Polysaccharid einen Substitutionsgrad von zwischen 0,05 und 1,0 und vorzugsweise von zwischen 0,2 und 0,5 aufweist.

4. Dialyselösung nach einem der Ansprüche 2 bis 3, **dadurch gekennzeichnet, dass** es sich bei der 2-Sulfoethylstärke zugrundeliegenden Stärke um eine Stärke handelt, die mit NaBH₄ vor der Sulfoethylierung reduziert wurde.

5. Dialyselösung nach einem der vorhergehenden Ansprüche, **dadurch gekennzeichnet, dass** das dem modifizierten Polysaccharid zugrundeliegende unmodifizierte Polysaccharid eine molare Masse zwischen 1.000-50.000 g/mol, vorzugsweise zwischen 1.000-20.000 g/mol aufweist und/oder dass die molare Masse der unmodifizierten Sulfoethylstärke zwischen 1.000 und 50.000 g/mol, vorzugsweise zwischen 1.000 und 20.000 g/mol liegt.

6. Dialyselösung nach einem der vorhergehenden Ansprüche, **dadurch gekennzeichnet, dass** die Dialyselösung außer dem modifizierten Polysaccharid kein weiteres Osmotikum enthält oder dass die Dialyselösung Mischungen aus dem modifizierten Polysaccharid und weiteren Osmotika und/oder Additiven wie z.B. L-Carnitin oder Taurin umfasst.

7. Dialyselösung nach einem der vorhergehenden Ansprüche, **dadurch gekennzeichnet, dass** die Dialyselösung ferner Elektrolyte und ein Puffersystem aufweist.

8. Dialyselösung nach Anspruch 7, **dadurch gekennzeichnet, dass** die Elektrolyte Natrium-, Kalium-, Calcium-, Magnesium- und/oder Chloridionen umfassen.

9. Dialyselösung nach Anspruch 7 oder 8, **dadurch gekennzeichnet, dass** es sich bei dem Puffersystem um einen Lactatpuffer, einen Hydrogencarbonatpuffer oder eine Kombination daraus handelt.

10. Dialyselösung nach einem der Ansprüche 7 bis 9, **dadurch gekennzeichnet, dass** die Elektrolyte, soweit vorhanden und unabhängig voneinander, in den folgenden Konzentrationen in der Dialyselösung vorliegen (Angaben in mmol/l):
| | |
|---|---|
| Natriumionen | 125-150 |
| Kaliumionen | 0-4,5 |
| Calciumionen | 0-2,5 |
| Magnesiumionen | 0-5 |
| Chloridionen | 90-120 |
| Lactat/Milchsäure/(Hydrogen)Carbonat/CO₂ | 30-60 |

11. Dialyselösung nach einem der vorhergehenden Ansprüche, **dadurch gekennzeichnet, dass** der pH-Wert der Dialyselösung bei Verwendung eines Einkammerbeutelssystems zwischen 5.0 und 8.0 und vorzugsweise zwischen 5.5 und 6.5 liegt und dass der pH-Wert der aus den in den Kammern eines Mehrkammerbeutels aufgenommenen Teillösungen hergestellten Dialyselösungen zwischen 5.0 und 8.0 und vorzugsweise zwischen 6.5 und 7.5 liegt.

12. Dialyselösung nach einem der vorhergehenden Ansprüche zur Anwendung in der Peritonealdialyse.

13. Verfahren zur Herstellung einer Dialyselösung nach einem der vorhergehenden Ansprüche umfassend den Schritt einer Modifikation eines Polysaccharids mit 2-Sulfoethylgruppen.

## Claims

1. A dialysis solution having at least one osmotic agent,
**characterized in that**
the osmotic agent is a polysaccharide that is modified by 2-sulfoethyl groups.

2. A dialysis solution in accordance with claim 1, **characterized in that** the modified polysaccharide is a 2-sulfoethyl starch.

3. A dialysis solution in accordance with one of the preceding claims, **characterized in that** the modified polysaccharide has a degree of substitution of between 0.05 and 1.0 and preferably of between 0.2 and 0.5.

4. A dialysis solution in accordance with one of the claims 2 to 3, **characterized in that** the starch underlying the 2-sulfoethyl starch is a starch that was reduced with NaBH₄ before the sulfoethylation.

5. A dialysis solution in accordance with one of the preceding claims, **characterized in that** the unmodified polysaccharide underlying the modified polysaccharide has a molar mass between 1,000 and 50,000 g/mol, preferably between 1,000 and 20,000 g/mol; and/or **in that** the molar mass of the unmodified sulfoethyl starch is between 1,000 and 50,000 g/mol, preferably between 1,000 and 20,000 g/mol.

6. A dialysis solution in accordance with one of the preceding claims, **characterized in that** the dialysis solution does not contain any further osmotic agent in addition to the modified polysaccharide; or **in that** the dialysis solution comprises mixtures of the modified polysaccharide and further osmotic agents and/or additives such as L-carnitine or taurine.

7. A dialysis solution in accordance with one of the preceding claims, **characterized in that** the dialysis solution furthermore has electrolytes and a buffer system.

8. A dialysis solution in accordance with claim 7, **characterized in that** the electrolytes comprise sodium ions, potassium ions, calcium ions, magnesium ions and/or chloride ions.

9. A dialysis solution in accordance with claim 7 or claim 8, **characterized in that** the buffer system is a lactate buffer, a hydrogen carbonate buffer, or a combination thereof.

10. A dialysis solution in accordance with one of the claims 7 to 9, **characterized in that** the electrolytes, where present and independently of one another, are present in the following concentrations in the dialysis solution (figures in mmol/l):
| | |
|---|---|
| Sodium ions | 125-150 |
| Potassium ions | 0-4.5 |
| Calcium ions | 0-2.5 |
| Magnesium ions | 0-5 |
| Chloride ions | 90-120 |
| Lactate/Lactic acid/(Hydrogen) carbonate/CO₂ | 30-60 |

11. A dialysis solution in accordance with one of the preceding claims, **characterized in that** the pH of the dialysis solution is between 5.0 and 8.0 and preferably between 5.5 and 6.5 on a use of a single-chamber bag system; and **in that** the pH of the dialysis solutions prepared from the partial solutions received in the chambers of a multi-chamber bag is between 5.0 and 8.0 and preferably between 6.5 and 7.5.

12. A dialysis solution in accordance with one of the preceding claims for use in peritoneal dialysis.

13. A method of preparing a dialysis solution in accordance with one of the preceding claims comprising the step of a modification of a polysaccharide by 2-sulfoethyl groups.

## Revendications

1. Solution de dialyse comprenant au moins un osmotique,
**caractérisée en ce que**
l'osmotique est un polysaccharide, qui est modifié avec des groupes 2-sulfoéthyle.

2. Solution de dialyse selon la revendication 1, **caractérisée en ce que** le polysaccharide modifié est un amidon 2-sulfoéthylique.

3. Solution de dialyse selon l'une des revendications précédentes, **caractérisée en ce que** le polysaccharide modifié présente un degré de substitution compris entre 0,05 et 1,0 et de préférence entre 0,2 et 0,5.

4. Solution de dialyse selon l'une des revendications 2 à 3, **caractérisée en ce que** l'amidon servant de base pour l'amidon 2-sulfoéthylique est un amidon qui a été réduit avec du NaBH₄ avant la sulfoéthylation.

5. Solution de dialyse selon l'une des revendications précédentes, **caractérisée en ce que** le polysaccharide non modifié servant de base pour le polysaccharide modifié présente une masse molaire comprise entre 1 000 et 50 000 g/mol, de préférence entre 1 000 et 20 000 g/mol et/ou **en ce que** la masse molaire de l'amidon sulfoéthylique non modifié est comprise entre 1 000 et 50 000 g/mol, de préférence entre 1 000 et 20 000 g/mol.

6. Solution de dialyse selon l'une des revendications précédentes, **caractérisée en ce que** la solution de dialyse ne contient pas d'autre osmotique en plus du polysaccharide modifié ou **en ce que** la solution de dialyse comprend des mélanges du polysaccharide modifié et d'autres osmotiques et/ou d'additifs tels que la L-carnitine ou la taurine.

7. Solution de dialyse selon l'une des revendications précédentes, **caractérisée en ce que** la solution de dialyse comporte en outre des électrolytes et un système de tampon.

8. Solution de dialyse selon la revendication 7, **caractérisée en ce que** les électrolytes comprennent des ions sodium, des ions potassium, des ions calcium, des ions magnésium et/ou des ions chlorure.

9. Solution de dialyse selon la revendication 7 ou 8, **caractérisée en ce que** le système de tampon est un tampon de lactate, un tampon d'hydrogénocarbonate ou une combinaison de ceux-ci.

10. Solution de dialyse selon l'une des revendications 7 à 9, **caractérisée en ce que** les électrolytes, le cas échéant et indépendamment les uns des autres, sont présents dans la solution de dialyse dans les concentrations suivantes (indications en mmol/l) :
| | |
|---|---|
| ions sodium | 125 à 150 |
| ions potassium | 0 à 4,5 |
| ions calcium | 0 à 2,5 |
| ions magnésium | 0 à 5 |
| ions chlorure | 90 à 120 |
| lactate/acide lactique/(hydrogéno)carbonate/CO₂ | 30 à 60. |

11. Solution de dialyse selon l'une des revendications précédentes, **caractérisée en ce que** le pH de la solution de dialyse, en cas d'utilisation d'un système de poche à un compartiment, est compris entre 5,0 et 8,0 et de préférence entre 5,5 et 6,5 et **en ce que** le pH des solutions de dialyse fabriquées à partir des solutions partielles reçues dans les compartiments d'une poche à compartiments multiples est compris entre 5,0 et 8,0 et de préférence entre 6,5 et 7,5.

12. Solution de dialyse selon l'une des revendications précédentes destinée à être utilisée en dialyse péritonéale.

13. Procédé de fabrication d'une solution de dialyse selon l'une des revendications précédentes, comprenant l'étape d'une modification d'un polysaccharide avec des groupes 2-sulfoéthyle.
